# EUROPÄISCHE PATENTANMELDUNG

(11) **EP 4 714 482 A1**
(43) Veröffentlichungstag der Anmeldung: **25.03.2026**
(21) Anmeldenummer: 25203037.4
(22) Anmeldetag: 18.09.2025
(51) Int. Cl.: A61M 16/00

(54) **STEUEREINHEIT FÜR EIN BEATMUNGSGERÄT**

(30) Priorität: 23.09.2024 DE 102024127439
(71) Anmelder: Conscientus ApS, 49900 Sakskøbing (DK)
(72) Erfinder: Kremeier, Peter, 76149 Karlsruhe (DE); Tusman, Gerardo, 7600 Mar del Plata (AR)
(74) Vertreter: Löwenstein Medical IP

(57) **Zusammenfassung**

Ein Beatmungsgerät (1) umfasst einen Atemluftanschluss (3) zum Anschließen des Atemapparats (5) eines Patienten, sodass eine Beatmung des Patienten mit Atemluft möglich ist. Des Weiteren umfasst das Beatmungsgerät (1) eine Aktorik (7) zum Bereitstellen eines Atemluftstroms (9) am Atemluftanschluss (3). Eine Steuereinheit (17) ist konfiguriert, um folgendes Verfahren auszuführen, wenn der Atemapparat (5) an den Atemluftanschluss (3) angeschlossen ist: Erzeugen eines Steuersignals (23) zum Steuern der Aktorik (7), sodass mindestens eine für die Beatmung relevante Istgröße, die einen Druck und/oder ein Volumen der Atemluft umfasst, bei jedem Atemzug (I, II, III) einem Sollverlauf (25) zwischen einer unteren Grenze (27) und einer oberen Grenze (29) folgt, wobei der Sollverlauf (25) in einer Einatmungsphase, in der der Patient einatmen soll, von der unteren Grenze auf die obere Grenze ansteigt und in einer Ausatmungsphase, in der der Patient ausatmen soll, von der oberen Grenze auf die untere Grenze abfällt; Umschalten der unteren Grenze (27) zwischen einem ersten Sollwert (v1) und einem zweiten Sollwert (v2), der betragsmäßig kleiner als der erste Sollwert ist, entsprechend einer sich zyklisch wiederholenden Atemzugreihenfolge, wobei die Atemzugreihenfolge in jeder Wiederholung einen oder mehrere erste Atemzüge (I), bei denen die mindestens eine Istgröße in der Ausatmungsphase auf den ersten Sollwert (v1) abfallen soll, und einen oder mehrere zweite Atemzüge (II), bei denen die mindestens eine Istgröße in der Ausatmungsphase auf den zweiten Sollwert (v2) abfallen soll, umfasst, wobei jedem zweiten Atemzug (II) oder jeder Sequenz von unmittelbar aufeinanderfolgenden zweiten Atemzügen ein erster Atemzug (I) oder eine Sequenz von unmittelbar aufeinanderfolgenden ersten Atemzügen (I) unmittelbar vorangeht.

## Beschreibung

### Technisches Gebiet

Die Erfindung betrifft eine Steuereinheit für ein Beatmungsgerät. Des Weiteren betrifft die Erfindung ein durch die Steuereinheit ausführbares Computerprogramm, ein entsprechendes computerlesbares Medium und ein Beatmungsgerät mit einer solchen Steuereinheit.

### Stand der Technik

Bei der künstlichen Beatmung spielt das sogenannte Totraumvolumen eine wichtige Rolle. Darunter kann im Allgemeinen derjenige Anteil des Atemzugvolumens verstanden werden, der bei jedem Atemzug innerhalb eines anatomischen und instrumentellen Totraums verbleibt (auch Totraumventilation genannt) und somit nicht am alveolären Gasaustausch teilnimmt. Der Totraum wird in jeder Ausatmungsphase mit Kohlendioxid befüllt, das in der folgenden Einatmungsphase wieder eingeatmet wird. Aufgrund des Totraums kann es dazu kommen, dass Kohlendioxid in unerwünschtem Maß im Körper zurückgehalten wird, was in manchen Fällen eine Hyperkapnie oder eine respiratorische Azidose verursachen kann. Beispielsweise kann bei ARDS- und COPD-Patienten (ARDS = akutes Atemnotsyndrom; COPD = chronisch obstruktive Lungenerkrankung) die Totraumventilation mindestens die Hälfte der Gesamtventilation pro Zeiteinheit betragen. Dies kann die Effizienz der Beatmung signifikant verringern. Prinzipiell kann eine Verringerung der Totraumventilation, d. h. eine Verbesserung der Kohlendioxidelimination, schützend für die Lunge wirken, weil dies die Verwendung eines geringeren Tidalvolumens und/oder eines geringeren Atemwegdrucks ermöglicht.

Der Totraum kann beispielsweise dadurch verringert werden, dass die den Atemapparat mit dem Beatmungsgerät verbindenden Komponenten in ihrer Anzahl und/oder ihrem Volumen verringert werden. Eine derartige Totraumverringerung lässt sich in der Praxis jedoch nicht ohne Weiteres umsetzen. Darüber hinaus ist es möglich, die Kohlendioxidelimination mittels einer geeigneten extrakorporalen Methode wie beispielsweise der extrakorporalen Membranoxygenierung, kurz ECMO, oder der extrakorporalen Kohlendioxidelimination, kurz ECCO₂R, zu steigern. Diese Methoden sind jedoch hochinvasiv und technisch sehr aufwendig.

### Offenbarung der Erfindung

Eine Aufgabe der Erfindung kann darin gesehen werden, eine Steuereinheit bereitzustellen, die es ermöglicht, die Kohlendioxidelimination bei der Beatmung eines Patienten in einfacher und schonender Weise zu verbessern. Eine weitere Aufgabe der Erfindung kann darin gesehen werden, ein entsprechendes Computerprogramm, ein entsprechendes computerlesbares Medium und ein entsprechendes Beatmungsgerät bereitzustellen.

Diese Aufgaben werden durch die Gegenstände der unabhängigen Ansprüche gelöst. Vorteilhafte Ausführungsformen der Erfindung sind in den abhängigen Ansprüchen, der nachfolgenden Beschreibung und den beigefügten Figuren dargelegt.

Ein erster Aspekt der Erfindung betrifft eine Steuereinheit für ein Beatmungsgerät. Das Beatmungsgerät umfasst einen Atemluftanschluss zum Anschließen des Atemapparats eines Patienten, sodass eine Beatmung des Patienten mit Atemluft möglich ist. Des Weiteren umfasst das Beatmungsgerät eine Aktorik zum Bereitstellen eines Atemluftstroms am Atemluftanschluss. Die Steuereinheit ist konfiguriert, um folgendes Verfahren auszuführen, wenn der Atemapparat an den Atemluftanschluss angeschlossen ist: Erzeugen eines Steuersignals zum Steuern der Aktorik, sodass mindestens eine für die Beatmung relevante Istgröße, die einen Druck und/oder ein Volumen der Atemluft umfasst, bei jedem Atemzug einem Sollverlauf zwischen einer unteren Grenze und einer oberen Grenze folgt, wobei der Sollverlauf in einer Einatmungsphase, in der der Patient einatmen soll, von der unteren Grenze auf die obere Grenze ansteigt und in einer Ausatmungsphase, in der der Patient ausatmen soll, von der oberen Grenze auf die untere Grenze abfällt; (bei der Beatmung und/oder beim Steuern der Aktorik:) Umschalten der unteren Grenze zwischen einem ersten Sollwert und einem zweiten Sollwert, der betragsmäßig kleiner als der erste Sollwert ist, entsprechend einer sich zyklisch wiederholenden Atemzugreihenfolge, wobei die Atemzugreihenfolge in jeder Wiederholung einen oder mehrere erste Atemzüge, bei denen die mindestens eine Istgröße in der (jeweiligen) Ausatmungsphase auf den ersten Sollwert abfallen soll, und einen oder mehrere zweite Atemzüge, bei denen die mindestens eine Istgröße in der (jeweiligen) Ausatmungsphase auf den zweiten Sollwert abfallen soll, umfasst, wobei jedem zweiten Atemzug oder jeder Sequenz von unmittelbar aufeinanderfolgenden zweiten Atemzügen ein erster Atemzug oder eine Sequenz von unmittelbar aufeinanderfolgenden ersten Atemzügen unmittelbar vorangeht.

Auf diese Weise kann eine gezielte periodische Schwankung der funktionellen Residualkapazität, kurz FRC, über einen oder mehrere Atemzüge hinweg bewirkt werden. Dies kann sich wiederum günstig auf die Kohlendioxidelimination auswirken. Insbesondere ermöglicht eine derartige Steuerung der jeweiligen beatmungsrelevanten Größe(n) eine signifikante Steigerung der durchschnittlichen Kohlendioxidelimination, ohne dass dadurch zwangsläufig das vom Patienten ausgeatmete Volumen zunimmt. Eine derartige Zunahme des Tidalvolumens sollte im Interesse einer möglichst schonenden Beatmung vermieden werden.

Die untere Grenze sollte in jedem Fall so gewählt werden, dass ein Lungenkollaps beim Ausatmen vermieden wird. Gleichzeitig sollte die Differenz zwischen dem ersten Sollwert und dem zweiten Sollwert groß genug gewählt werden, damit eine therapeutisch wirksame Steigerung der Kohlendioxidelimination erzielt werden kann.

Die untere Grenze kann beispielsweise einen unteren Solldruck umfassen, auf den der Druck der Atemluft in jeder Ausatmungsphase abfallen soll. In entsprechender Weise kann die obere Grenze einen oberen Solldruck umfassen. Der untere Solldruck kann auch als positiver endexspiratorischer Druck, kurz PEEP, bezeichnet werden. Der untere Solldruck kann als ein betragsmäßig kleinerer Druck als der obere Solldruck aufgefasst werden.

Zusätzlich oder alternativ kann die untere Grenze ein unteres Sollvolumen umfassen, auf den das Volumen der Atemluft in jeder Ausatmungsphase abfallen soll. In entsprechender Weise kann die obere Grenze ein oberes Sollvolumen umfassen. Das untere Sollvolumen kann als ein betragsmäßig kleineres Volumen als das obere Sollvolumen aufgefasst werden.

Es ist möglich, dass das Beatmungsgerät ferner eine Sensorik zum Erzeugen von Messdaten bezüglich der Beatmung umfasst (siehe auch weiter unten). Die Sensorik kann beispielsweise einen Drucksensor zum Erfassen des Drucks der Atemluft und/oder einen Flusssensor zum Erfassen eines Volumenstroms der Atemluft umfassen. In diesem Fall kann das Verfahren ferner umfassen: Empfangen der Messdaten, wobei die Messdaten einen oder mehrere (bei der Beatmung erfasste) Werte für die Istgröße(n) umfassen. Dementsprechend ist es möglich, dass das Steuersignal unter Verwendung der Messdaten erzeugt wird, insbesondere derart, dass sich die jeweilige Istgröße, genauer deren Verlauf, durch entsprechendes Steuern der Aktorik dem Sollverlauf annähert. Die Messdaten können zusätzlich oder alternativ zum Anpassen des Sollverlaufs (beispielsweise des ersten Sollwerts und/oder des zweiten Sollwerts) und/oder der Atemzugreihenfolge verwendet werden.

Bei dem Druck der Atemluft kann es sich im weiteren Sinn um einen Druck der Atemluft in der Lunge des Patienten handeln. In entsprechender Weise kann es sich bei dem Volumen der Atemluft im weiteren Sinn um ein luftgefülltes Volumen der Lunge handeln.

Es ist möglich, dass mindestens eine Wiederholung der Atemzugreihenfolge von mindestens einer anderen Wiederholung der Atemzugreihenfolge abweicht, beispielsweise in der Anzahl und/oder Art und/oder Reihenfolge der Atemzüge. Anders ausgedrückt kann die Atemzugreihenfolge bei der Beatmung von Wiederholung zu Wiederholung variiert werden. Dies ermöglicht eine Anpassung der Atemzugreihenfolge an Änderungen des Zustands des Patienten. Alternativ kann die Atemzugreihenfolge in jeder Wiederholung die gleiche sein.

Die verschiedenen Wiederholungen der Atemzugreihenfolge können zumindest teilweise unmittelbar aufeinanderfolgen. Dabei kann dem ersten Atemzug in einer aktuellen Wiederholung der letzte Atemzug in einer früheren Wiederholung, die der aktuellen Wiederholung unmittelbar vorangeht, unmittelbar vorangehen und/oder dem letzten Atemzug in einer aktuellen Wiederholung der erste Atemzug in einer späteren Wiederholung, die der aktuellen Wiederholung unmittelbar folgt, unmittelbar folgen.

Beispielsweise kann die Atemzugreihenfolge in mindestens einer oder jeder Wiederholung neben einem zweiten Atemzug eine Sequenz von mindestens zwei oder mindestens vier unmittelbar aufeinanderfolgenden ersten Atemzügen umfassen, wobei dem zweiten Atemzug die Sequenz der ersten Atemzüge unmittelbar vorangehen kann.

Das Verfahren kann beispielsweise computerimplementiert sein.

Die Steuereinheit kann Mittel zur Datenverarbeitung umfassen. Die Mittel zur Datenverarbeitung können als Hard- und/oder Software implementiert sein und/oder einen Prozessor umfassen. Der Prozessor kann konfiguriert sein, um das (computerimplementierte) Verfahren auszuführen. Zusätzlich zum Prozessor kann die Steuereinheit mindestens eines der folgenden Mittel zur Datenverarbeitung umfassen: einen Speicher, ein Bussystem zur Datenkommunikation zwischen dem Speicher und dem Prozessor, eine Datenkommunikationsschnittstelle zur drahtlosen und/oder drahtgebundenen Datenkommunikation mit Peripheriegeräten. Alternativ kann die Steuereinheit ausschließlich als Hardware, beispielsweise in Form eines ASIC- oder FPGA-Bausteins, implementiert sein.

Ein zweiter Aspekt der Erfindung betrifft ein Beatmungsgerät. Das Beatmungsgerät umfasst einen Atemluftanschluss zum Anschließen des Atemapparats eines Patienten, sodass eine Beatmung des Patienten mit Atemluft möglich ist. Des Weiteren umfasst das Beatmungsgerät eine Aktorik zum Bereitstellen eines Atemluftstroms am Atemluftanschluss. Zudem umfasst das Beatmungsgerät eine Steuereinheit, wie sie vor- und nachstehend beschrieben wird. Die Steuereinheit kann beispielsweise in ein Gehäuse des Beatmungsgeräts integriert sein. Alternativ ist eine externe Steuereinheit möglich.

Unter "Beatmungsgerät" kann beispielsweise ein Gerät zum invasiven und/oder nicht invasiven Beatmen des Patienten und/oder ein Anästhesiegerät verstanden werden.

Der Atemluftanschluss kann über einen oder mehrere Beatmungsschläuche und/oder über eine geeignete Patientenschnittstelle wie beispielsweise eine Maske, eine Nasenkanüle oder einen Tubus an den Atemapparat anschließbar sein.

Die Aktorik kann einen oder mehrere elektropneumatische Aktoren umfassen. Beispielsweise kann die Aktorik ein oder mehrere Gebläse und/oder ein oder mehrere elektrisch steuerbare Ventile umfassen.

Ein dritter Aspekt der Erfindung betrifft ein Computerprogramm zum Betreiben eines Beatmungsgeräts, wie es vor- und nachstehend beschrieben wird. Das Computerprogramm umfasst Befehle, die die Steuereinheit - beispielsweise einen Prozessor der Steuereinheit - beim Ausführen des Computerprogramms durch die Steuereinheit veranlassen, folgendes (computerimplementiertes) Verfahren auszuführen, wenn der Atemapparat an den Atemluftanschluss angeschlossen ist: Erzeugen eines Steuersignals zum Steuern der Aktorik, sodass mindestens eine für die Beatmung relevante Istgröße, die einen Druck und/oder ein Volumen der Atemluft umfasst, bei jedem Atemzug einem Sollverlauf zwischen einer unteren Grenze und einer oberen Grenze folgt, wobei der Sollverlauf in einer Einatmungsphase, in der der Patient einatmen soll, von der unteren Grenze auf die obere Grenze ansteigt und in einer Ausatmungsphase, in der der Patient ausatmen soll, von der oberen Grenze auf die untere Grenze abfällt; (bei der Beatmung und/oder beim Steuern der Aktorik:) Umschalten der unteren Grenze zwischen einem ersten Sollwert und einem zweiten Sollwert, der betragsmäßig kleiner als der erste Sollwert ist, entsprechend einer sich zyklisch wiederholenden Atemzugreihenfolge, wobei die Atemzugreihenfolge in jeder Wiederholung einen oder mehrere erste Atemzüge, bei denen die mindestens eine Istgröße in der (jeweiligen) Ausatmungsphase auf den ersten Sollwert abfallen soll, und einen oder mehrere zweite Atemzüge, bei denen die mindestens eine Istgröße in der (jeweiligen) Ausatmungsphase auf den zweiten Sollwert abfallen soll, umfasst, wobei jedem zweiten Atemzug oder jeder Sequenz von unmittelbar aufeinanderfolgenden zweiten Atemzügen ein erster Atemzug oder eine Sequenz von unmittelbar aufeinanderfolgenden ersten Atemzügen unmittelbar vorangeht.

Ein vierter Aspekt der Erfindung betrifft ein computerlesbares Medium, auf dem ein Computerprogramm, wie es vor- und nachstehend beschrieben wird, gespeichert ist.

Das computerlesbare Medium kann ein flüchtiger oder nicht flüchtiger Datenspeicher sein. Beispielsweise kann das computerlesbare Medium eine Festplatte, ein USB-Speichergerät (USB = *universal serial bus*), ein RAM (*random-access memory*), ein ROM (*read-only memory*), ein EPROM (*erasable programmable read-only memory*), ein EEPROM (*electrically erasable programmable read-only memory*), ein Flash-Speicher oder eine Kombination aus mindestens zwei dieser Beispiele sein. Das computerlesbare Medium kann auch ein Datenkommunikationsnetzwerk, das das Herunterladen von Programmcode ermöglicht (z. B. über das Internet), oder eine Cloud sein.

Es wird darauf hingewiesen, dass Merkmale der vor- und nachstehend beschriebenen Steuereinheit auch Merkmale des Computerprogramms und/oder des computerlesbaren Mediums sein können (und umgekehrt).

Im Folgenden werden verschiedene Ausführungsformen der Erfindung beschrieben. Diese Ausführungsformen sind nicht als Beschränkung des Umfangs der Erfindung zu verstehen.

Gemäß einer Ausführungsform kann der Ausatmungsphase eines jeden ersten Atemzugs ein abfallender erster Abschnitt des Sollverlaufs zugeordnet sein. In entsprechender Weise kann der Ausatmungsphase eines jeden zweiten Atemzugs ein abfallender zweiter Abschnitt des Sollverlaufs zugeordnet sein. Die ersten Abschnitte und die zweiten Abschnitte können in ihrer jeweiligen Dauer und/oder Amplitude in jeder Wiederholung der Atemzugreihenfolge so aufeinander abgestimmt sein, dass ein vom Patienten bei jedem zweiten Atemzug ausgeatmetes Volumen höchstens so groß wie ein vom Patienten bei jedem ersten Atemzug ausgeatmetes Volumen ist. Anders ausgedrückt kann der Sollverlauf so definiert sein, dass sich das Atemzug- oder Tidalvolumen des Patienten von Atemzug zu Atemzug nicht maßgeblich ändert, insbesondere bei jedem Übergang von einem ersten Atemzug zu einem zweiten Atemzug nicht maßgeblich zunimmt. Somit kann eine möglichst schonende Beatmung gewährleistet werden. Unter "Amplitude" kann eine Differenz zwischen der jeweiligen oberen Grenze und der jeweiligen unteren Grenze (auf den die jeweilige Istgröße in der Ausatmungsphase abfallen soll) verstanden werden.

Gemäß einer Ausführungsform kann das Verfahren ferner umfassen: Empfangen eines Dehnbarkeitswerts, der eine Dehnbarkeit zumindest eines Teils des Atemapparats des Patienten anzeigt, und/oder eines Widerstandswerts, der einen Strömungswiderstand in zumindest einem Teil des Atemapparats des Patienten anzeigt; Bestimmen der jeweiligen Dauer und/oder Amplitude der ersten Abschnitte und/oder der zweiten Abschnitte unter Verwendung des Dehnbarkeitswerts und/oder des Widerstandswerts. Beispielsweise kann der Dehnbarkeitswert eine gemessene und/oder geschätzte Compliance der Lunge des Patienten und/oder kann der Widerstandswert eine gemessene und/oder geschätzte Resistance der Atemwege des Patienten anzeigen. Dies ermöglicht eine einfache Anpassung der jeweiligen Abschnitte des Sollverlaufs an verschiedene physiologische Zustände des Atemapparats im Hinblick auf eine möglichst schonende Beatmung.

Gemäß einer Ausführungsform kann ein Produkt durch Multiplizieren des Dehnbarkeitswerts und des Widerstandswerts berechnet werden. Das Produkt kann dann zum Bestimmen der jeweiligen Dauer und/oder Amplitude der ersten Abschnitte und/oder der zweiten Abschnitte verwendet werden. Bei dem Produkt kann es sich beispielsweise um eine Zeitkonstante handeln. Anders ausgedrückt können der Dehnbarkeitswert und der Widerstandswert durch Berechnen ihres Produkts in einem einzelnen Wert zusammengefasst werden. Dies kann nachfolgende Berechnungsschritte in der Steuereinheit vereinfachen.

Gemäß einer Ausführungsform kann die jeweilige Dauer der ersten Abschnitte und/oder der zweiten Abschnitte umso länger gewählt werden, je größer der Betrag des Produkts ist. Zusätzlich oder alternativ kann die jeweilige Amplitude der ersten Abschnitte und/oder der zweiten Abschnitte umso größer gewählt werden, je größer der Betrag des Produkts ist. Dies ermöglicht eine einfache Skalierung der Dauer bzw. der Amplitude abhängig vom jeweiligen Zustand des Atemapparats.

Zusätzlich oder alternativ kann der Dehnbarkeitswert und/oder kann der Widerstandswert und/oder kann das Produkt aus dem Dehnbarkeitswert und dem Widerstandswert zum Bestimmen einer Anzahl der zweiten Atemzüge in jeder Wiederholung der Atemzugreihenfolge und/oder zum Bestimmen eines Verhältnisses einer Anzahl der ersten Atemzüge zu einer Anzahl der zweiten Atemzüge in jeder Wiederholung der Atemzugreihenfolge verwendet werden.

Gemäß einer Ausführungsform kann das Verfahren ferner umfassen: Empfangen eines Differenzwerts für jeden Atemzug; Bestimmen eines aktuellen Werts der oberen Grenze für den jeweiligen Atemzug durch Addieren des Differenzwerts zu einem aktuellen Wert der unteren Grenze. Es ist möglich, dass für jeden Atemzug der gleiche Differenzwert empfangen wird. Alternativ können für verschiedene Atemzüge zumindest teilweise verschiedene Differenzwerte empfangen werden. Der Differenzwert kann beispielsweise unter Berücksichtigung eines maximalen Beatmungsdrucks und/oder -volumens, mit dem der Patient in der Einatmungsphase höchstens beatmet werden darf, festgelegt worden sein. Bei dem aktuellen Wert der unteren Grenze kann es sich je nach Typ des aktuellen Atemzugs entsprechend der Atemzugreihenfolge um den ersten Sollwert, den zweiten Sollwert oder einen sonstigen Sollwert, beispielsweise einen dritten Sollwert (siehe weiter unten), handeln.

Gemäß einer Ausführungsform kann das Verfahren ferner umfassen: Empfangen eines alternativen Differenzwerts für jeden Atemzug, der einem zweiten Atemzug unmittelbar folgt; Bestimmen eines aktuellen Werts der oberen Grenze für den jeweiligen Atemzug, der dem zweiten Atemzug unmittelbar folgt, durch Addieren des alternativen Differenzwerts statt des Differenzwerts zu einem aktuellen Wert der unteren Grenze. Die untere Grenze in der Einatmungsphase des jeweiligen Atemzugs, der dem zweiten Atemzug unmittelbar folgt, ist entsprechend der vorangegangenen Ausatmungsphase normalerweise auf den zweiten (niedrigeren) Sollwert gesetzt. Diese Absenkung kann beim Bestimmen der oberen Grenze für den jeweiligen Atemzug mithilfe eines entsprechend angepassten alternativen Differenzwerts in geeigneter Weise berücksichtigt, beispielsweise ausgeglichen werden.

Anders ausgedrückt kann die Differenz zwischen der unteren Grenze und der oberen Grenze je nach Atemzug in Bezug auf die Ein- und/oder Ausatmungsphase gezielt variiert werden. Beispielsweise kann der Differenzwert für jeden Atemzug, der einem zweiten Atemzug unmittelbar folgt, um einen bestimmten Betrag größer als für jeden Atemzug, der einem zweiten Atemzug unmittelbar vorangeht, gewählt werden, insbesondere derart, dass die obere Grenze für diese beiden Atemzüge gleich groß ist. Alternativ kann für jeden Atemzug der gleiche Differenzwert empfangen werden. In diesem Fall kann die obere Grenze in ihrem Wert entsprechend (beispielsweise periodisch) schwanken.

Gemäß einer Ausführungsform kann der alternative Differenzwert gleich einer Summe sein, die sich durch Addieren des Differenzwerts zu einer Differenz zwischen dem ersten Sollwert und dem zweiten Sollwert ergibt. Dadurch kann erreicht werden, dass die obere Grenze in jedem ersten Atemzug, der einem zweiten Atemzug unmittelbar folgt, die gleiche ist wie in jedem ersten Atemzug, der einem zweiten Atemzug unmittelbar vorangeht.

Gemäß einer Ausführungsform kann ein Verhältnis einer Anzahl der ersten Atemzüge zu einer Anzahl der zweiten Atemzüge in jeder Wiederholung der Atemzugreihenfolge und/oder pro Zeiteinheit, beispielsweise pro Minute, mindestens zwei zu eins betragen. Das Verhältnis kann fest vorgegeben sein oder bei der Beatmung je nach Zustand des Patienten von Wiederholung zu Wiederholung variiert werden. Beispielsweise kann das Verhältnis der Anzahl der ersten Atemzüge zur Anzahl der zweiten Atemzüge pro Zeiteinheit drei zu eins, vier zu eins, fünf zu eins oder mehr als fünf zu eins betragen. In manchen Fällen kann auch ein Verhältnis von eins zu eins zweckmäßig sein.

Gemäß einer Ausführungsform kann die Ausatmungsphase eines jeden zweiten Atemzugs höchstens 3 Sekunden, vorzugsweise höchstens 1 Sekunde, dauern. Bei einer derartigen Dauer kann erfahrungsgemäß eine ausreichend wirksame Steigerung der (durchschnittlichen) Kohlendioxidelimination erreicht werden, ohne dass dadurch das Tidalvolumen oder das Risiko eines Lungenkollapses maßgeblich zunimmt.

Gemäß einer Ausführungsform kann der zweite Sollwert zwischen 20 und 70 Prozent des ersten Sollwerts betragen. Ein Verhältnis in diesem Prozentbereich hat sich in der Praxis als besonders günstig erwiesen.

Gemäß einer Ausführungsform kann die untere Grenze entsprechend der Atemzugreihenfolge zwischen dem ersten Sollwert, dem zweiten Sollwert und einem zwischen dem ersten Sollwert und dem zweiten Sollwert liegenden dritten Sollwert umgeschaltet werden. In diesem Fall kann die Atemzugreihenfolge in mindestens einer (oder jeder) Wiederholung ferner einen oder mehrere dritte Atemzüge umfassen, bei denen die mindestens eine Istgröße in der (jeweiligen) Ausatmungsphase auf den dritten Sollwert abfallen soll. Dabei kann jedem ersten Atemzug, der einem zweiten Atemzug oder einer Sequenz von unmittelbar aufeinanderfolgenden zweiten Atemzügen unmittelbar vorangeht, oder jeder Sequenz von ersten Atemzügen, die einem zweiten Atemzug oder einer Sequenz von unmittelbar aufeinanderfolgenden zweiten Atemzügen unmittelbar vorangeht, ein dritter Atemzug oder eine Sequenz von unmittelbar aufeinanderfolgenden dritten Atemzügen unmittelbar vorangehen. Bei dem dritten Sollwert kann es sich beispielsweise um einen Referenzwert, insbesondere in Form eines normalen PEEP-Werts, handeln, der für einen überwiegenden Anteil oder zumindest für die Hälfte der Atemzüge pro Wiederholung oder pro Zeiteinheit verwendet werden soll. Dadurch, dass die untere Grenze ausgehend von diesem Referenzwert zunächst auf den ersten Sollwert angehoben wird, bevor sie in der nachfolgenden Ausatmungsphase auf den zweiten Sollwert abgesenkt wird, kann die alveoläre Ventilation weiter verbessert werden.

Beispielsweise kann die Atemzugreihenfolge in mindestens einer oder jeder Wiederholung neben einem ersten Atemzug und einem zweiten Atemzug eine Sequenz von mindestens zwei oder mindestens vier unmittelbar aufeinanderfolgenden dritten Atemzügen umfassen, wobei dem zweiten Atemzug der erste Atemzug und dem ersten Atemzug die Sequenz der dritten Atemzüge unmittelbar vorangehen kann.

Gemäß einer Ausführungsform kann der dritte Sollwert zwischen 50 und 80 Prozent des ersten Sollwerts betragen. Ein Verhältnis in diesem Prozentbereich hat sich in der Praxis als besonders günstig erwiesen.

Gemäß einer Ausführungsform kann das Beatmungsgerät ferner eine Sensorik zum Erzeugen von Messdaten bezüglich der Beatmung umfassen. In diesem Fall kann das Verfahren ferner ein Empfangen der Messdaten in mehreren aufeinanderfolgenden Zeitschritten umfassen. Dabei können die Messdaten mindestens einen der folgenden Datentypen umfassen: Volumendaten, die ein vom Patienten ausgeatmetes Kohlendioxidvolumen (beispielsweise ein Kohlendioxidminutenvolumen) anzeigen; Partialdruckdaten, die einen (beispielsweise endtidalen) Kohlendioxidpartialdruck in einer vom Patienten ausgeatmeten Atemluft und/oder im Blut des Patienten anzeigen; Bilddaten, die eine zwei- und/oder dreidimensionale Ausdehnung luftgefüllter Regionen der Lunge des Patienten anzeigen. Die Bilddaten können vorzugsweise mittels elektrischer Impedanztomografie, kurz EIT, erzeugt worden sein. Möglich sind aber auch andere invasive oder nicht invasive bildgebende Verfahren. Darüber hinaus kann das Verfahren folgenden Schritt umfassen: Bestimmen von Auswertungsdaten, die einen geschätzten Verlauf einer Kohlendioxidelimination bei der Beatmung anzeigen, unter Verwendung der Messdaten aus mindestens zwei Zeitschritten. Beispielsweise können die Auswertungsdaten in jedem Zeitschritt aus den Messdaten eines aktuellen Zeitschritts und mindestens eines dem aktuellen Zeitschritt vorangegangenen Zeitschritts bestimmt werden.

Zusätzlich kann das Verfahren folgenden Schritt oder folgende Schritte umfassen: Anzeigen der Auswertungsdaten auf einem Display und/oder Verwenden der Auswertungsdaten (und/oder der Messdaten) zum Erzeugen des Steuersignals und/oder zum Anpassen des Sollverlaufs (beispielsweise durch Ändern des ersten Sollwerts und/oder des zweiten Sollwerts und/oder einer Differenz zwischen dem ersten Sollwert und dem zweiten Sollwert), insbesondere derart, dass sich der geschätzte Verlauf der Kohlendioxidelimination einem gewünschten Wertebereich annähert.

### Kurze Beschreibung der Zeichnungen

Im Folgenden werden Ausführungsformen der Erfindung mit Bezug auf die beigefügten Zeichnungen beschrieben. Weder die Beschreibung noch die Zeichnungen sind als Beschränkung des Umfangs der Erfindung zu verstehen.
Fig. 1 zeigt ein Beatmungsgerät gemäß einer Ausführungsform der Erfindung.
Fig. 2 zeigt einen Solldruckverlauf entsprechend einer sich zyklisch wiederholenden Atemzugreihenfolge von ersten und zweiten Atemzügen zur Verwendung in einem Verfahren, das durch eine Steuereinheit gemäß einer Ausführungsform der Erfindung ausgeführt werden kann.
Fig. 3 zeigt einen Solldruckverlauf entsprechend einer sich zyklisch wiederholenden Atemzugreihenfolge von ersten, zweiten und dritten Atemzügen zur Verwendung in einem Verfahren, das durch eine Steuereinheit gemäß einer Ausführungsform der Erfindung ausgeführt werden kann.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen der Erfindung

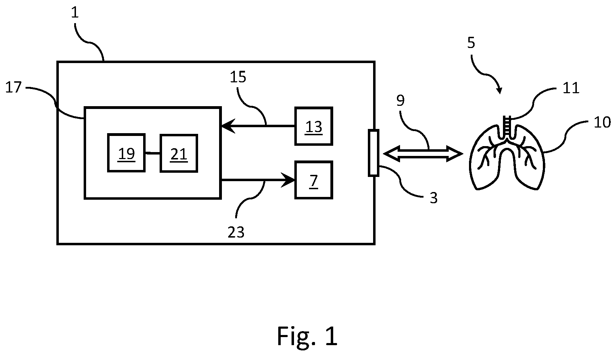

Fig. 1 zeigt ein Beatmungsgerät 1 zum invasiven und/oder nicht invasiven Beatmen eines Patienten. Das Beatmungsgerät 1 umfasst einen Atemluftanschluss 3, an den der Atemapparat 5 des Patienten angeschlossen ist, sodass eine Beatmung des Patienten mit Atemluft möglich ist, und eine Aktorik 7 zum Bereitstellen eines Atemluftstroms 9 am Atemluftanschluss 3. Beispielsweise kann der Atemluftanschluss 3 über einen oder mehrere Beatmungsschläuche und eine geeignete Patientenschnittstelle wie eine Maske, eine Nasenkanüle oder einen Tubus an die Lunge 10 und/oder die Atemwege 11 des Patienten angeschlossen sein.

Zudem umfasst das Beatmungsgerät 1 eine Sensorik 13 zum Erzeugen von Messdaten 15 bezüglich der Beatmung. Die Sensorik 13 und die Aktorik 7 können jeweils an eine Steuereinheit 17 angeschlossen sein. Die Steuereinheit 17 kann ausgebildet sein, um die Aktorik 7 unter Verwendung der Messdaten 15 zu steuern.

Die Aktorik 7 kann ein oder mehrere Gebläse und/oder ein oder mehrere elektrische steuerbare Ventile umfassen.

Die Sensorik 13 kann einen oder mehrere Sensoren umfassen, beispielsweise mindestens einen der folgenden Sensoren: einen Kohlendioxidsensor zum Erfassen eines Kohlendioxidpartialdrucks in der Atemluft und/oder im Blut des Patienten; einen Sauerstoffsensor zum Erfassen eines Sauerstoffpartialdrucks in der Atemluft und/oder im Blut des Patienten; einen Flusssensor zum Erfassen eines Volumenstroms der Atemluft; einen Drucksensor zum Erfassen eines Drucks der Atemluft; eine Mehrzahl von Elektroden zum Messen von Änderungen eines elektrischen Widerstands im Lungengewebe des Patienten, beispielsweise im Rahmen einer elektrischen Impedanztomografie.

Dementsprechend können die Messdaten 15 mindestens einen der folgenden Datentypen umfassen: Volumendaten, die ein vom Patienten ausgeatmetes Kohlendioxid- und/oder Sauerstoffvolumen (beispielsweise jeweils als Minutenvolumen) anzeigen; Partialdruckdaten, die den (beispielsweise endtidalen) Kohlendioxid- und/oder Sauerstoffpartialdruck anzeigen; Bilddaten, die eine zwei- und/oder dreidimensionale Ausdehnung luftgefüllter Regionen der Lunge des Patienten anzeigen.

Die Bilddaten können beispielsweise eine Helligkeit und/oder Farbe für jeden Bildpunkt (auch Pixel genannt) einer zwei- oder dreidimensionalen Matrix von Bildpunkten in Bezug auf eine oder mehrere Querschnittsebenen der Lunge 10 codieren. Dabei kann die Helligkeit und/oder Farbe abhängig von einem dem jeweiligen Bildpunkt zugeordneten gemessenen elektrischen Widerstand variieren. Beispielsweise können jedem Bildpunkt drei Werte zwischen 0 und 100 Prozent oder zwischen 0 und 255 (bei 8 Bit) zur Codierung einer jeweiligen Helligkeit der Farben Rot, Grün und Blau zugeordnet sein. Möglich sind aber auch andere Farbräume und/oder andere Arten der Codierung.

Die Steuereinheit 17 kann einen Prozessor 19 und einen Speicher 21 umfassen, in dem ein Computerprogramm zum Betreiben des Beatmungsgeräts 1 gespeichert sein kann. Der Prozessor 19 kann konfiguriert sein, um durch Ausführen des Computerprogramms folgendes Verfahren zum Betreiben des Beatmungsgeräts 1 auszuführen.

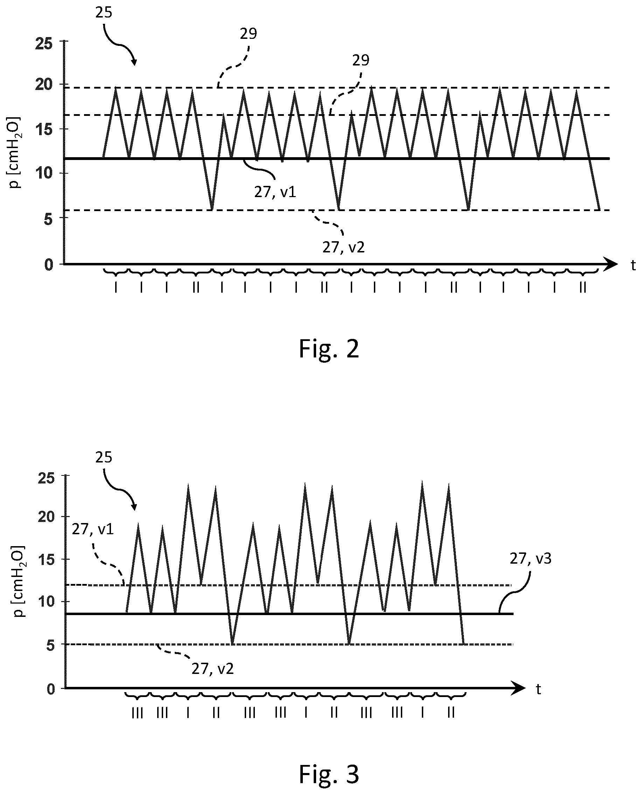

In einem ersten Schritt wird ein Steuersignal 23 zum Steuern der Aktorik 7 erzeugt, sodass mindestens eine für die Beatmung relevante Istgröße, die einen Druck p und/oder ein Volumen der Atemluft umfassen kann, bei jedem Atemzug einem Sollverlauf 25 zwischen einer unteren Grenze 27 und einer oberen Grenze 29 folgt (siehe Fig. 2 und Fig. 3). In diesem Beispiel ist die Istgröße ein Druck p der Atemluft. Der Sollverlauf 25 kann in einer Einatmungsphase, in der der Patient einatmen soll, von der unteren Grenze 27 (hier einem unteren Solldruck) auf die obere Grenze 29 (hier einen oberen Solldruck) ansteigen und in einer Ausatmungsphase, in der der Patient ausatmen soll, von der oberen Grenze 29 auf die untere Grenze 27 abfallen.

In einem zweiten Schritt wird die untere Grenze 27 zwischen einem ersten Sollwert v1 (hier einem ersten Solldruckwert) und einem zweiten Sollwert v2 (hier einem zweiten Solldruckwert), der betragsmäßig kleiner als der erste Sollwert v1 ist, entsprechend einer vorgegebenen, sich zyklisch wiederholenden Atemzugreihenfolge umgeschaltet. Die Atemzugreihenfolge kann in jeder Wiederholung einen oder mehrere erste Atemzüge I, bei denen der Druck p in der jeweiligen Ausatmungsphase auf den ersten Sollwert v1 abfallen soll, und einen oder mehrere zweite Atemzüge II, bei denen der Druck p in der jeweiligen Ausatmungsphase auf den zweiten Sollwert v2 abfallen soll, umfassen. Dabei kann jedem zweiten Atemzug II oder jeder Sequenz von unmittelbar aufeinanderfolgenden zweiten Atemzügen II ein erster Atemzug I oder eine Sequenz von unmittelbar aufeinanderfolgenden ersten Atemzügen I unmittelbar vorangehen.

Der erste Schritt und der zweite Schritt können gleichzeitig oder zeitlich zueinander versetzt ausgeführt werden.

In dem in Fig. 2 gezeigten Beispiel geht jedem zweiten Atemzug II eine Sequenz von vier (bzw. drei) unmittelbar aufeinanderfolgenden ersten Atemzügen I unmittelbar voran. Dementsprechend beträgt ein Verhältnis einer Anzahl der ersten Atemzüge I zu einer Anzahl der zweiten Atemzüge II in jeder Wiederholung, beispielsweise pro Minute, vier (bzw. drei) zu eins. Möglich sind aber auch andere Verhältnisse, beispielsweise von eins zu eins, zwei zu eins oder fünf zu eins. Vorzugweise beträgt der zweite Sollwert v2 zwischen 20 und 70 Prozent des ersten Sollwerts v1.

Wie in Fig. 3 gezeigt, kann die Atemzugreihenfolge in jeder Wiederholung ferner einen oder mehrere dritte Atemzüge III umfassen, bei denen der Druck p in der jeweiligen Ausatmungsphase auf einen dritten Sollwert v3 (hier einem dritten Solldruckwert) abfallen soll. Der dritte Sollwert v3 (hier 8 cmH₂O) kann betragsmäßig zwischen dem ersten Sollwert v1 (hier 12 cmH₂O) und dem zweiten Sollwert v2 (hier 5 cmH₂O) liegen. Vorzugsweise beträgt der dritte Sollwert v3 zwischen 50 und 80 Prozent des ersten Sollwerts v1.

Somit ist es möglich, dass die untere Grenze 27 gemäß der Atemzugreihenfolge zwischen dem ersten Sollwert v1, dem zweiten Sollwert v2 und dem dritten Sollwert v3 umgeschaltet wird. In diesem Beispiel geht jedem ersten Atemzug I, der einem zweiten Atemzug II unmittelbar vorangeht, eine Sequenz von zwei unmittelbar aufeinanderfolgenden dritten Atemzügen III unmittelbar voran. Dementsprechend beträgt ein Verhältnis zwischen einer Anzahl der dritten Atemzüge III, einer Anzahl der ersten Atemzüge I und einer Anzahl der zweiten Atemzüge II in jeder Wiederholung, beispielsweise pro Minute, zwei zu eins zu eins. Möglich sind aber auch andere geeignete Verhältnisse, beispielsweise von eins zu eins zu eins. Denkbar ist ferner, dass das Verhältnis bei der Beatmung je nach Zustand des Patienten fortlaufend variiert wird.

Bei dem dritten Sollwert v3 kann es sich beispielsweise um einen normalen PEEP-Wert handeln, der für einen überwiegenden Anteil oder zumindest für die Hälfte der Atemzüge pro Wiederholung oder pro Zeiteinheit verwendet wird. Dadurch, dass die untere Grenze 27 ausgehend vom normalen PEEP-Wert zunächst auf den ersten Sollwert v1 angehoben wird, bevor sie in der nachfolgenden Ausatmungsphase auf den zweiten Sollwert v2 abgesenkt wird, kann die alveoläre Ventilation weiter verbessert werden.

Es ist möglich, dass der Ausatmungsphase eines jeden ersten Atemzugs I ein abfallender erster Abschnitt des Sollverlaufs 25, der Ausatmungsphase eines jeden zweiten Atemzugs II ein abfallender zweiter Abschnitt des Sollverlaufs 25 und der Ausatmungsphase eines jeden dritten Atemzugs III ein abfallender dritter Abschnitt des Sollverlaufs 25 zugeordnet ist. Die jeweiligen Abschnitte der verschiedenen Atemzugtypen I, II, III können in ihrer jeweiligen Dauer und/oder Amplitude in jeder Wiederholung der Atemzugreihenfolge so aufeinander abgestimmt sein, dass ein vom Patienten bei jedem Atemzug ausgeatmetes Volumen über mehrere Atemzüge hinweg mehr oder weniger gleich bleibt, insbesondere bei jedem Übergang von einem ersten Atemzug I zu einem zweiten Atemzug II nicht maßgeblich zunimmt. Somit kann trotz einer im Schnitt deutlich gesteigerten Kohlendioxidelimination eine möglichst schonende Beatmung gewährleistet werden.

Um eine Anpassung der jeweiligen Dauer und/oder Amplitude - insbesondere der zweiten Abschnitte - an den jeweiligen Zustand des Patienten zu ermöglichen, kann in einem optionalen Schritt ein Dehnbarkeitswert, der eine Dehnbarkeit zumindest eines Teils des Atemapparats 5 (beispielsweise eine Compliance der Lunge 10) anzeigt, empfangen werden. Zusätzlich oder alternativ kann ein Widerstandswert, der einen Strömungswiderstand in zumindest einem Teil des Atemapparats 5 (beispielsweise eine Resistance der Atemwege 11) anzeigt, empfangen werden. Bei dem Dehnbarkeitswert und dem Widerstandswert kann es sich jeweils um einen (beispielsweise mittels eines oder mehrerer geeigneter Sensoren der Sensorik 13) gemessenen und/oder geschätzten Wert handeln.

Beispielsweise können der Dehnbarkeitswert und der Widerstandswert miteinander multipliziert werden. Das resultierende Produkt, beispielsweise in Form einer Zeitkonstante, kann dann zum Bestimmen der jeweiligen Dauer und/oder Amplitude der ersten und/oder zweiten Abschnitte verwendet werden. Dabei kann die Dauer umso länger gewählt werden, je größer der Betrag des Produkts ist. In entsprechender Weise kann die Amplitude umso größer gewählt werden, je größer der Betrag des Produkts ist. Dies ermöglicht eine einfache Skalierung der Dauer bzw. der Amplitude abhängig vom jeweiligen Zustand des Atemapparats 5.

Zusätzlich oder alternativ kann der Dehnbarkeitswert und/oder kann der Widerstandswert und/oder kann das Produkt aus dem Dehnbarkeitswert und dem Widerstandswert zum Bestimmen einer Anzahl der zweiten Atemzüge II in jeder Wiederholung der Atemzugreihenfolge und/oder zum Bestimmen eines Verhältnisses einer Anzahl der zweiten Atemzüge II zu einer Anzahl der ersten Atemzüge I und/oder zu einer Anzahl der dritten Atemzüge III in jeder Wiederholung der Atemzugreihenfolge verwendet werden.

In der Praxis hat sich eine Dauer von höchstens 1 bis 3 Sekunden für die Ausatmungsphase eines jeden zweiten Atemzugs II bewährt. Je nach Zustand des Patienten sind aber auch andere Werte möglich.

Die obere Grenze 29 kann in ihrem Wert beispielsweise dadurch bestimmt werden, dass für jeden einzelnen Atemzug I, II oder III ein geeigneter Differenzwert (hier ein geeigneter Druckdifferenzwert) empfangen wird und der Differenzwert dann zu einem aktuellen Wert v1, v2 oder v3 der unteren Grenze 27 addiert wird, um einen aktuellen Wert der oberen Grenze 29 für den jeweiligen Atemzug I, II oder III zu erhalten.

Der Differenzwert kann für jeden Atemzug der gleiche sein. Alternativ können für verschiedene Atemzüge zumindest teilweise verschiedene, d. h. in ihrem Betrag voneinander abweichende Differenzwerte empfangen werden.

Möglich ist beispielsweise, dass für jeden Atemzug I oder III, der einem zweiten Atemzug II unmittelbar folgt, ein alternativer Differenzwert (hier ein alternativer Druckdifferenzwert), der in seinem Betrag vom (normalen) Differenzwert in geeigneter Weise abweicht, beispielsweise betragsmäßig größer als der (normale) Differenzwert ist, empfangen wird. Der aktuelle Wert der oberen Grenze 29 für den jeweiligen Atemzug I oder III kann dann durch Addieren des alternativen Differenzwerts statt des (normalen) Differenzwerts zum jeweiligen aktuellen Wert der unteren Grenze 27, hier v2, bestimmt werden. Beispielsweise kann der alternative Differenzwert gleich einer Summe sein, die sich durch Addieren des (normalen) Differenzwerts zu einer Differenz zwischen dem ersten Sollwert v1 und dem zweiten Sollwert v2 ergibt.

Die Messdaten 15 können beispielsweise in mehreren aufeinanderfolgenden Zeitschritten empfangen werden, beispielsweise mit einer Frequenz zwischen 1 Hz und 1000 Hz, vorzugsweise zwischen 100 Hz und 300 Hz. Aus den Messdaten 15 verschiedener Zeitschritte können in einem weiteren optionalen Schritt Auswertungsdaten, die einen geschätzten, beispielsweise zeitabhängigen Verlauf der Kohlendioxidelimination bei der Beatmung anzeigen, bestimmt werden. Die Auswertungsdaten können dann auf einem Display, beispielsweise in Form von Zahlenwerten und/oder eines Diagramms, zu Überwachungszwecken angezeigt werden. Zusätzlich oder alternativ können die Auswertungsdaten zum Erzeugen des Steuersignals 23 und/oder zum Anpassen zumindest eines Abschnitts des Sollverlaufs 25, beispielsweise durch Ändern von mindestens einem der Sollwerte v1, v2 und v3, verwendet werden. Denkbar ist etwa, dass der Sollverlauf 25 (oder zumindest ein Abschnitt davon) in einem aktuellen Zeitschritt so angepasst wird, dass der geschätzte Verlauf der Kohlendioxidelimination in einem zukünftigen Zeitschritt in einem gewünschten Wertebereich liegt oder zumindest dem gewünschten Wertebereich im zukünftigen Zeitschritt näher als im aktuellen Zeitschritt ist.

Bekanntermaßen ist die funktionelle Residualkapazität, kurz FRC, beim gesunden Menschen nicht konstant, sondern schwankt mit niedrigen zyklischen Frequenzen von etwa 1 zu 500 Atemzügen, was sich in entsprechender Weise auf den Gasaustausch auswirkt. Der vorstehend beschriebene Beatmungsmodus berücksichtigt dieses physiologische Verhalten, beschleunigt jedoch solche Schwankungen, um eine zusätzliche Kohlendioxidelimination bei der mechanischen Beatmung zu erreichen. Dazu können alle paar Atemzüge wiederkehrende, kurze und kontrollierte Reduktionen der funktionellen Residualkapazität vorgenommen werden. Der Beatmungsmodus kann daher auch als Beatmung mit variierbarer FRC bezeichnet werden. Ein solcher Beatmungsmodus kann als unabhängiger Beatmungsmodus in einem Beatmungsgerät oder in Verbindung mit anderen Vorrichtungen verwendet werden, um anatomische und instrumentelle Toträume aktiv zu reduzieren.

Der Beatmungsmodus kann in einer automatischen zyklischen FRC-Verringerung gegenüber einem Basiswert bestehen, indem beispielsweise der PEEP-Wert für die Dauer eines einzelnen Atemzugs gesenkt wird. Somit ist die PEEP-Absenkung besonders kurz, beispielsweise kürzer als 1 Sekunde. Eine solche PEEP-Absenkung kann beispielsweise alle zwei bis vier Atemzüge erfolgen. Die zusätzliche FRC-Senkung bewirkt eine zusätzliche Elimination von Kohlendioxid aus den kleineren Atemwegen und dem alveolären Kompartiment. Da das Kohlendioxid dieser speziellen Atemzüge überwiegend aus den Alveolen stammt, wird die alveoläre Ventilation ohne Totraum in den Atemwegen verstärkt.

Die FRC-Verringerung sollte sehr kurz sein, um einen Lungenkollaps zu vermeiden. Generell sollte die Dauer der FRC-Verringerung deutlich unter der Zeitkonstante für einen Atemweg- und/oder Alveolarkollaps liegen. Nach einer Reihe normaler Atemzüge bei einem Basis-PEEP sollte das PEEP-Niveau für einen einzelnen Atemzug reduziert werden, sodass die FRC abnehmen kann. Diese vorübergehende FRC-Verringerung kann bereits während der anschließenden Einatmungsphase kompensiert werden. Das Beatmungsgerät sollte daher einen ausreichend hohen Volumenstrom für die Einatmung bereitstellen können, damit nicht nur die FRC wiederhergestellt wird, sondern auch das gewünschte Tidalvolumen bereitgestellt wird.

Beispielsweise kann der Beatmungsmodus so konfiguriert sein, dass der PEEP-Wert von einem Atemzug zum nächsten um ungefähr 5 cmH₂O verringert oder erhöht wird, während ein ausreichend hoher Atemluftstrom in der Einatmungsphase bereitgestellt wird, sodass jede Änderung der FRC schnell ausgeglichen werden kann und eine normale Tidalventilation aufrechterhalten wird. Die Druckdifferenz zwischen oberem und unterem Solldruck sollte unterhalb von 15 mmHg liegen, um eine möglichst lungenschonende Beatmung zu ermöglichen.

Die FRC-Änderungen können in ihrer Anzahl pro Zeiteinheit, beispielsweise pro Minute, und/oder in ihrer Amplitude manuell eingestellt werden. Alternativ kann die Einstellung automatisch durch das Beatmungsgerät erfolgen.

Es ist möglich, dass der Beatmungsmodus automatisch eine Folge von FRC-Änderungen bei konstantem Tidalvolumen abhängig vom jeweiligen Differenzwert erzeugt. Dabei umfasst das insgesamt ausgeatmete Atemminutenvolumen einen ersten Teil, der dem Produkt aus Tidalvolumen und Atemfrequenz entspricht, und einen zweiten Teil, der dem aufgrund der zusätzlichen PEEP-Absenkung zusätzlich ausgeatmeten Volumen entspricht. Beispielsweise kann ein Standard-Atemminutenvolumen von 6,75 l/min (Produkt aus einem Tidalvolumen von 450 ml und einer Atemfrequenz von 15/min) zusammen mit einer wiederholten kurzen PEEP-Absenkung zunächst von 10 cmH₂O auf 5 cmH₂O und dann wieder auf 10 cmH₂O vorgegeben werden. Unter der Annahme, dass eine solche PEEP-Absenkung eine zusätzliche Ausatmung von 200 ml bei jedem vierten Atemzug verursacht, kann eine zusätzliche Beatmung von etwa 1 l/min (200 ml mal 5 Atemzüge pro Minute) erreicht werden, was ein Gesamt-Atemminutenvolumen von 7,75 l/min ergibt. Mit anderen Worten: Je geringer das Verhältnis zwischen normalen Atemzügen und speziellen Atemzügen (mit abgesenktem PEEP) ist und je größer die PEEP-Absenkung ist, desto größer ist der Effekt der zusätzlichen Kohlendioxidelimination.

Mithilfe einer Atemzugreihenfolge, wie sie in Fig. 3 beispielhaft veranschaulicht ist, kann die Amplitude der FRC-Änderung zusätzlich vergrößert werden und die Alveolarventilation entsprechend verbessert werden. Dabei kann die untere Grenze 27 beispielsweise regelmäßig um plus/minus 3 bis 4 cmH₂O um einen PEEP-Basiswert schwanken. Der PEEP-Basiswert sollte so eingestellt sein, dass die Lunge beim Ausatmen nicht kollabiert. Bei einem PEEP-Basiswert von 8 cmH₂O und einem Verhältnis von eins zu eins zu eins könnte die untere Grenze 27 in ihrem Wert beispielsweise folgendermaßen zyklisch schwanken: 8-12-4-8-12-4-8-12-4 usw. (8-8-12-4-8-8-12-4-8-8-12-4 usw. bei einem Verhältnis von zwei zu eins zu eins). Eine derartige PEEP-Absenkung, hier jeweils um 8 cmH₂O, kann die Kohlendioxidelimination signifikant steigern. Dies ermöglicht es, das Tidalvolumen bei schwankender FRC auf einen Mindestwert zu reduzieren.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

### Liste der Bezugszeichen

- 1: Beatmungsgerät
- 3: Atemluftanschluss
- 5: Atemapparat
- 7: Aktorik
- 9: Atemluftstrom
- 10: Lunge
- 11: Atemwege
- 13: Sensorik
- 15: Messdaten
- 17: Steuereinheit
- 19: Prozessor
- 21: Speicher
- 23: Steuersignal
- 25: Sollverlauf
- 27: untere Grenze
- 29: obere Grenze
- p: Druck
- t: Zeit
- v1: erster Sollwert
- v2: zweiter Sollwert
- v3: dritter Sollwert
- I: erster Atemzug
- II: zweiter Atemzug
- III: dritter Atemzug

## Patentansprüche

1. Steuereinheit (17) für ein Beatmungsgerät (1), wobei das Beatmungsgerät (1) umfasst:
einen Atemluftanschluss (3) zum Anschließen des Atemapparats (5) eines Patienten, sodass eine Beatmung des Patienten mit Atemluft möglich ist;
eine Aktorik (7) zum Bereitstellen eines Atemluftstroms (9) am Atemluftanschluss (3);
wobei die Steuereinheit (17) konfiguriert ist, um folgendes Verfahren auszuführen, wenn der Atemapparat (5) an den Atemluftanschluss (3) angeschlossen ist:
Erzeugen eines Steuersignals (23) zum Steuern der Aktorik (7), sodass mindestens eine für die Beatmung relevante Istgröße, die einen Druck (p) und/oder ein Volumen der Atemluft umfasst, bei jedem Atemzug (I, II, III) einem Sollverlauf (25) zwischen einer unteren Grenze (27) und einer oberen Grenze (29) folgt, wobei der Sollverlauf (25) in einer Einatmungsphase, in der der Patient einatmen soll, von der unteren Grenze (27) auf die obere Grenze (29) ansteigt und in einer Ausatmungsphase, in der der Patient ausatmen soll, von der oberen Grenze (29) auf die untere Grenze (27) abfällt;
Umschalten der unteren Grenze (27) zwischen einem ersten Sollwert (v1) und einem zweiten Sollwert (v2), der betragsmäßig kleiner als der erste Sollwert (v1) ist, entsprechend einer sich zyklisch wiederholenden Atemzugreihenfolge, wobei die Atemzugreihenfolge in jeder Wiederholung einen oder mehrere erste Atemzüge (I), bei denen die mindestens eine Istgröße in der Ausatmungsphase auf den ersten Sollwert (v1) abfallen soll, und einen oder mehrere zweite Atemzüge (II), bei denen die mindestens eine Istgröße in der Ausatmungsphase auf den zweiten Sollwert (v2) abfallen soll, umfasst, wobei jedem zweiten Atemzug (II) oder jeder Sequenz von unmittelbar aufeinanderfolgenden zweiten Atemzügen (II) ein erster Atemzug (I) oder eine Sequenz von unmittelbar aufeinanderfolgenden ersten Atemzügen (I) unmittelbar vorangeht.

2. Steuereinheit (17) nach Anspruch 1,
wobei der Ausatmungsphase eines jeden ersten Atemzugs (I) ein abfallender erster Abschnitt des Sollverlaufs (25) zugeordnet ist und der Ausatmungsphase eines jeden zweiten Atemzugs (II) ein abfallender zweiter Abschnitt des Sollverlaufs (25) zugeordnet ist, wobei die ersten Abschnitte und die zweiten Abschnitte in ihrer jeweiligen Dauer und/oder Amplitude in jeder Wiederholung der Atemzugreihenfolge so aufeinander abgestimmt sind, dass ein vom Patienten bei jedem zweiten Atemzug (II) ausgeatmetes Volumen höchstens so groß wie ein vom Patienten bei jedem ersten Atemzug (I) ausgeatmetes Volumen ist.

3. Steuereinheit (17) nach Anspruch 2,
wobei das Verfahren ferner umfasst:
Empfangen eines Dehnbarkeitswerts, der eine Dehnbarkeit zumindest eines Teils (10, 11) des Atemapparats (5) des Patienten anzeigt, und/oder eines Widerstandswerts, der einen Strömungswiderstand in zumindest einem Teil (10, 11) des Atemapparats (5) des Patienten anzeigt;
Bestimmen der jeweiligen Dauer und/oder Amplitude der ersten Abschnitte und/oder der zweiten Abschnitte unter Verwendung des Dehnbarkeitswerts und/oder des Widerstandswerts.

4. Steuereinheit (17) nach Anspruch 3,
wobei ein Produkt durch Multiplizieren des Dehnbarkeitswerts und des Widerstandswerts bestimmt wird und das Produkt zum Bestimmen der jeweiligen Dauer und/oder Amplitude verwendet wird.

5. Steuereinheit (17) nach Anspruch 4,
wobei die jeweilige Dauer umso länger gewählt wird, je größer der Betrag des Produkts ist; und/oder
wobei die jeweilige Amplitude umso größer gewählt wird, je größer der Betrag des Produkts ist.

6. Steuereinheit (17) nach einem der vorhergehenden Ansprüche,
wobei das Verfahren ferner umfasst:
Empfangen eines Differenzwerts für jeden Atemzug (I, II, III);
Bestimmen eines aktuellen Werts der oberen Grenze (29) für den jeweiligen Atemzug (I, II, III) durch Addieren des Differenzwerts zu einem aktuellen Wert (v1, v2, v3) der unteren Grenze (27).

7. Steuereinheit (17) nach Anspruch 6,
wobei das Verfahren ferner umfasst:
Empfangen eines alternativen Differenzwerts für jeden Atemzug (I, III), der einem zweiten Atemzug (II) unmittelbar folgt;
Bestimmen eines aktuellen Werts der oberen Grenze (29) für den jeweiligen Atemzug (I, III), der dem zweiten Atemzug (II) unmittelbar folgt, durch Addieren des alternativen Differenzwerts statt des Differenzwerts zu einem aktuellen Wert (v2) der unteren Grenze (27).

8. Steuereinheit (17) nach Anspruch 7,
wobei der alternative Differenzwert gleich einer Summe ist, die sich durch Addieren des Differenzwerts zu einer Differenz zwischen dem ersten Sollwert (v1) und dem zweiten Sollwert (v2) ergibt.

9. Steuereinheit (17) nach einem der vorhergehenden Ansprüche,
wobei ein Verhältnis einer Anzahl der ersten Atemzüge (I) zu einer Anzahl der zweiten Atemzüge (II) in jeder Wiederholung der Atemzugreihenfolge mindestens zwei zu eins beträgt; und/oder
wobei die Ausatmungsphase eines jeden zweiten Atemzugs (II) höchstens 3 Sekunden, vorzugsweise höchstens 1 Sekunde, dauert; und/oder
wobei der zweite Sollwert (v2) zwischen 20 und 70 Prozent des ersten Sollwerts (v1) beträgt.

10. Steuereinheit (17) nach einem der vorhergehenden Ansprüche,
wobei die untere Grenze (27) entsprechend der Atemzugreihenfolge zwischen dem ersten Sollwert (v1), dem zweiten Sollwert (v2) und einem zwischen dem ersten Sollwert (v1) und dem zweiten Sollwert (v2) liegenden dritten Sollwert (v3) umgeschaltet wird, wobei die Atemzugreihenfolge in mindestens einer Wiederholung ferner einen oder mehrere dritte Atemzüge (III) umfasst, bei denen die mindestens eine Istgröße in der Ausatmungsphase auf den dritten Sollwert (v3) abfallen soll, wobei jedem ersten Atemzug (I), der einem zweiten Atemzug (II) oder einer Sequenz von unmittelbar aufeinanderfolgenden zweiten Atemzügen (II) unmittelbar vorangeht, oder jeder Sequenz von ersten Atemzügen (I), die einem zweiten Atemzug (II) oder einer Sequenz von unmittelbar aufeinanderfolgenden zweiten Atemzügen (II) unmittelbar vorangeht, ein dritter Atemzug (III) oder eine Sequenz von unmittelbar aufeinanderfolgenden dritten Atemzügen (III) unmittelbar vorangeht.

11. Steuereinheit (17) nach einem der vorhergehenden Ansprüche,
wobei das Beatmungsgerät (1) ferner eine Sensorik (13) zum Erzeugen von Messdaten (15) bezüglich der Beatmung umfasst, wobei das Verfahren ferner umfasst:
Empfangen der Messdaten (15) in mehreren aufeinanderfolgenden Zeitschritten, wobei die Messdaten (15) mindestens einen der folgenden Datentypen umfassen: Volumendaten, die ein vom Patienten ausgeatmetes Kohlendioxidvolumen anzeigen; Partialdruckdaten, die einen Kohlendioxidpartialdruck in einer vom Patienten ausgeatmeten Atemluft und/oder im Blut des Patienten anzeigen; Bilddaten, die eine zwei- und/oder dreidimensionale Ausdehnung luftgefüllter Regionen der Lunge (9) des Patienten anzeigen;
Bestimmen von Auswertungsdaten, die einen geschätzten Verlauf einer Kohlendioxidelimination bei der Beatmung anzeigen, unter Verwendung der Messdaten (15) aus mindestens zwei Zeitschritten.

12. Steuereinheit (17) nach Anspruch 11,
wobei das Verfahren ferner umfasst:
Verwenden der Auswertungsdaten zum Erzeugen des Steuersignals (23) und/oder zum Anpassen des Sollverlaufs (25), sodass sich der geschätzte Verlauf der Kohlendioxidelimination einem gewünschten Wertebereich annähert.

13. Beatmungsgerät (1), umfassend:
einen Atemluftanschluss (3) zum Anschließen des Atemapparats (5) eines Patienten, sodass eine Beatmung des Patienten mit Atemluft möglich ist;
eine Aktorik (7) zum Bereitstellen eines Atemluftstroms (9) am Atemluftanschluss (3);
eine Steuereinheit (17) nach einem der vorhergehenden Ansprüche.

14. Computerprogramm zum Betreiben des Beatmungsgeräts (1) nach Anspruch 13, wobei das Computerprogramm Befehle umfasst, die die Steuereinheit (17) beim Ausführen des Computerprogramms durch die Steuereinheit (17) veranlassen, folgendes Verfahren auszuführen, wenn der Atemapparat (5) an den Atemluftanschluss (3) angeschlossen ist:
Erzeugen eines Steuersignals (23) zum Steuern der Aktorik (7), sodass mindestens eine für die Beatmung relevante Istgröße, die einen Druck (p) und/oder ein Volumen der Atemluft umfasst, bei jedem Atemzug (I, II, III) einem Sollverlauf (25) zwischen einer unteren Grenze (27) und einer oberen Grenze (29) folgt, wobei der Sollverlauf (25) in einer Einatmungsphase, in der der Patient einatmen soll, von der unteren Grenze (27) auf die obere Grenze (29) ansteigt und in einer Ausatmungsphase, in der der Patient ausatmen soll, von der oberen Grenze (29) auf die untere Grenze (27) abfällt;
Umschalten der unteren Grenze (27) zwischen einem ersten Sollwert (v1) und einem zweiten Sollwert (v2), der betragsmäßig kleiner als der erste Sollwert (v1) ist, entsprechend einer sich zyklisch wiederholenden Atemzugreihenfolge, wobei die Atemzugreihenfolge in jeder Wiederholung einen oder mehrere erste Atemzüge (I), bei denen die mindestens eine Istgröße in der Ausatmungsphase auf den ersten Sollwert (v1) abfallen soll, und einen oder mehrere zweite Atemzüge (II), bei denen die mindestens eine Istgröße in der Ausatmungsphase auf den zweiten Sollwert (v2) abfallen soll, umfasst, wobei jedem zweiten Atemzug (II) oder jeder Sequenz von unmittelbar aufeinanderfolgenden zweiten Atemzügen (II) ein erster Atemzug (I) oder eine Sequenz von unmittelbar aufeinanderfolgenden ersten Atemzügen (I) unmittelbar vorangeht.

15. Computerlesbares Medium, auf dem das Computerprogramm nach Anspruch 14 gespeichert ist.
